# EUROPEAN PATENT SPECIFICATION

(11) **EP 4 147 649 B1**
(45) Date of publication and mention of the grant of the patent: **21.10.2026**
(21) Application number: 22205655.8
(22) Date of filing: 10.02.2016
(51) Int. Cl.: A61B 17/00

(54) **CLOSURE DEVICE FOR SEALING PERCUTANEOUS OPENING IN A VESSEL**
VERSCHLUSSVORRICHTUNG ZUM VERSCHLIESSEN EINER PERKUTANEN ÖFFNUNG IN EINEM GEFÄSS
DISPOSITIF DE FERMETURE POUR FERMER UNE OUVERTURE PERCUTANÉE DANS UN VAISSEAU

(30) Priority: 10.02.2015 US 201562114101 P
(43) Date of publication of application: 15.03.2023
(62) Document number(s) of the earlier application(s) in accordance with Art. 76 EPC: 16712553.3 / 3 256 051
(73) Proprietor: Teleflex Life Sciences LLC, Wilmington, DE 19808 (US)
(72) Inventor: JACOBS, Peter, Minneapolis, 55369 (US); HOLMAN, Thomas, Minneapolis, 55369 (US); KUGLER, Chad, Minneapolis, 55369 (US)
(74) Representative: Marks & Clerk LLP

(56) References cited:
- EP-A1- 2 497 426
- EP-A2- 2 258 258
- CA-C- 2 238 784
- US-A1- 2002 099 448
- US-A1- 2008 147 038
- US-A1- 2013 190 810

## Description

### TECHNICAL FIELD

This patent document relates to medical devices. More particularly, but not by way of limitation, the patent document relates to closure systems.

### BACKGROUND

Catheterization and interventional procedures, such as angioplasty and stenting, are generally performed by inserting a hollow needle through a patient's skin and any intervening tissue into a blood vessel of the vascular system. A guidewire can then be passed through a lumen of the needle into the blood vessel accessed by the needle. The needle can be removed, and an introducer sheath in conjunction with, or subsequent to, a dilator can be advanced over the guidewire and into the vessel. The introducer sheath can facilitate introducing various devices into the vessel, while minimizing trauma to the vessel wall or minimizing blood loss during a procedure. For example, a catheter can be advanced through a lumen of the introducer sheath and over the guidewire into a position for performing an interventional procedure.

Upon completion of the interventional procedure, for example, the catheter and introducer sheath can be removed, leaving a puncture in the vessel wall. The puncture tends to bleed, particularly in the case of arterial punctures because of the higher arterial blood pressure as compared to venous blood pressure. Until the puncture is closed, clinical complications can result leading to increased hospital stays and costs. To address this concern, medical personnel are required to provide constant and continuing care to patients who have undergone an interventional procedure involving an arterial or venous puncture to ensure that post-operative bleeding is controlled.

A common method of controlling a puncture in a vessel wall is to maintain external pressure (e.g., human hand pressure) over the vessel until the puncture seals by natural clot formation processes. This method of puncture closure typically takes between 30 and 90 minutes, with the length of time being greater if the patient is hypertensive or anti-coagulated. Utilizing external pressure to control bleeding can suffer from several drawbacks regardless of whether the patient is hypertensive or anti-coagulated. For example, human hand pressure can be uncomfortable for the patient, can result in excessive restriction or interruption of blood flow, and can consume costly time and effort on the part of the hospital staff. Other pressure techniques, such as pressure bandages, sandbags and clamps can also suffer from drawbacks, including requiring the patient to remain motionless for an extended period of time and requiring close monitoring of the patient by hospital staff to ensure effectiveness of these techniques.

EP 2497426A1 discloses a device for closing a puncture in a body lumen. The device includes a support structure, a tight patch member attached to the support structure, and an attachment unit. The aggregate has a first shape for insertion into the lumen and second shape for when the aggregate is inside the body lumen.

US 2002/099448A1 describes a medical device comprising a collagenous biomaterial, such as a submucosa of a tissue.

US 2008/147038A1 describes a lesion site marking device and a method for marking a lesion site using the device. The device includes a filler body, a marker, which is encapsulated in a covering.

CA 2238784A1 discloses a temporary bioabsorbable-radiopaque marker for use in an implantable endoprosthesis, such as a stent.

EP 2258258A2 discloses a biopsy localization device that includes a bioabsorbable element that is insertable into the target location via a delivery device.

US 2013/190810A1 discloses a closure device for closing an opening in the wall of a body lumen. The closure device includes a closure element with a proximal flange and a distal flange and a first coupler of the proximal flange that connects to a second coupler of the distal flange.

WO 2007/149989A2 proposes medical graft products, systems, and methods useful for treating fistulae, particularly enterocutaneous fistulae.

### OVERVIEW

The present inventors recognize that an ever-expanding range of catheterization and interventional procedures and a changing reimbursement landscape, with an increasing adoption of outpatient interventions, drive the need for more efficient puncture closure at the end of procedures. The present inventors further recognize that with an ever-increasing number of procedures requiring large introducer sheaths, such as abdominal aortic aneurysm repair, thoracic aneurysm repair, transcutaneous aortic valve implantation (TAVI), trans-septal occluder implantation and implantation of a variety of percutaneous ventricular-assist devices, the ability to achieve closure following sheath removal is increasingly important. According to existing techniques, when large introducer sheaths are used during a percutaneous procedure, a surgical cut-down is often performed to expose the femoral artery and a labor-intensive suture procedure is used to establish vessel wall closure.

The present closure systems, kits and methods can be used to seal a percutaneous puncture or other opening in a blood vessel wall, body cavity or biopsy tract. The present teachings have particular relevance to reliably and consistently sealing a puncture access site opening in a vessel following a TAVI procedure or delivery or use of another large profile interventional device. The teachings can eliminate the prolonged bleeding associated with such punctures, prevent disposing any occlusive material into the vessel, prevent introducing infectious organisms into a patient's circulatory system, and avoid labor-intensive external pressure procedures on the part of hospital staff.

According to the present invention, there is provided a closure system as set out in claim 1.

The one or more radiopaque elements may be water soluble, and/or may be coated on the inner member body. Alternatively, the one or more radiopaque elements may be within the inner member body.

The first end region me be about one-third (1/3) longer than the second end region.

A first radiopaque element of the one or more radiopaque elements may be encapsulated within the first end region, and a second radiopaque element of the one or more radiopaque elements may be encapsulated within the second end region.

The length of the inner member body may range from about 10 mm to about 20 mm, inclusive, and the width at the enlarged region may range from about 4 mm to about 8 mm, inclusive.

The inner member body may have a size and shape that does not block or otherwise impede a flow of blood through the vessel wall when the inner member abuts the inner surface of the vessel wall and overlays the percutaneous puncture. For example, the inner member may be composed of a thin, narrow strip or bar of material that is sufficiently rigid to be resistant to deformation, yet thin enough to conform to the inner surface of the vessel wall and not take up a substantial portion of the lumen of the vessel

The closure system may further comprise a connecting member that is movably coupled to the inner member body. The connecting member may a stem, rod, tube, string, thread or other filament, and/or may be movably coupled to the inner member body at a location closer to the second end of the inner member body than the first end of the inner member body.

The closure system may further comprise an outer member expandable from a delivery configuration to a sealing configuration, the sealing configuration having a longitudinally-compressed and transversely-expanded state relative to the delivery configuration. The outer member may be bioabsorbable.

The closure system may further comprise a locking member securable to the connecting member and configured to provide resistance to a proximal end of the outer member from retracting proximally.

These and other examples and features of the present systems will be set forth, at least in part, in the following Detailed Description. This Overview is intended to provide non-limiting examples of the present teachings-it is not intended to provide an exclusive or exhaustive explanation. The Detailed Description below is included to provide further information about the present systems.

### BRIEF DESCRIPTION OF THE DRAWINGS

In the drawings, like numerals can be used to describe similar features and components throughout the several views. The drawings illustrate generally, by way of example but not by way of limitation, various embodiments discussed in this patent document.
- FIG. **1**: is a schematic illustration of a punctured wall of a blood vessel, such as an artery.
- FIG. **2**: is a schematic illustration of an implant assembly sealing a punctured wall of a blood vessel, as constructed in accordance with at least one embodiment of the present teachings.
- FIG. **3**: is an exploded illustration of an implant assembly, as constructed in accordance with at least one embodiment of the present teachings.
- FIGS. **4** and **5**: are schematic illustrations of an inner member and a connecting member in a deployed orientation, as constructed in accordance with at least two embodiments of the present teachings.
- FIG. **6**: is a schematic illustration of a sealing membrane attached to an inner member and surrounding portions of a connecting member, as constructed in accordance with at least one embodiment of the present teachings.
- FIGS. **7A** and **7B**: are schematic illustrations of an outer member receivable within a sealing membrane and expandable from a delivery configuration to a sealing configuration, as constructed in accordance with at least one embodiment of the present teachings.
- FIGS. **8A, 8B** and **9**: are schematic illustrations of a locking member that can be received by a proximal end of a connecting member, as constructed in accordance with at least two embodiments of the present teachings.
- FIG. **10A**: is a schematic cross-sectional illustration of a proximal portion of a delivery assembly configured to deliver and deploy an implant assembly at a puncture site, as constructed in accordance with at least one embodiment.
- FIG. **10B**: is a schematic cross-sectional illustration of an implant assembly mounted on a distal portion of a delivery assembly, as constructed in accordance with at least one embodiment.
- FIGS. **11A-11C**: are schematic illustrations, in sequential order, of an introducer sheath and a delivery assembly delivering and deploying an implant assembly at a puncture site, as constructed in accordance with at least one embodiment of the present teachings.
- FIG. **12**: is a schematic illustration of a kit including an implant assembly, a delivery assembly, an introducer sheath and a valve bypass, a dilator, and instructions for using these components to seal a puncture site or other opening in a blood vessel wall, body cavity or biopsy tract, as constructed in accordance with at least one embodiment of the present teachings.

The drawing figures are not necessarily to scale. Certain features and components may be shown exaggerated in scale or in schematic form, and some details may not be shown in the interest of clarity and conciseness.

### DETAILED DESCRIPTION

A closure system can comprise an implant assembly, a delivery assembly and an introducer sheath. The closure system can further comprise a valve bypass and a dilator. The implant assembly can include an inner member, a sealing membrane, and an outer member, each of which can be delivered by the delivery assembly. The inner member can be extended through the puncture or opening and positioned adjacent an inner tissue surface. The outer member can be positioned adjacent an outer tissue surface. The sealing membrane can have a distal end attached to the inner member, a proximal end including an opening configured to receive the outer member, and a mid-region therebetween. The outer member, when expanded from a delivery configuration to a sealing configuration, can urge the mid-region of the sealing membrane radially outward such that its outer surface can contact and conform to an edge of the puncture or opening.

The delivery assembly for delivery and deploying the implant assembly can comprise a handle, a rail, a shear tube, a delivery tube, and an actuation member. The handle can have a first housing portion and a second housing portion. The rail can extend from a first end engaged with the inner member to a second end statically coupled with the second housing portion. The outer member can be supported by the rail between its first and second ends. The shear tube can extend from a first end, which includes a keyed passageway, to a second end engaged with the second housing portion. The delivery tube can concentrically surround portions of the shear tube, be coupled to an end of the sealing membrane on its first end, and be coupled to the first housing portion on its second end. The actuation member can be engaged with the second end of the shear tube to urge the tube in a direction to expand the outer member from its delivery configuration to its sealing configuration.

A method for sealing a puncture , which is not part of the invention, that extends between an inner vessel surface and an outer vessel surface can comprise inserting an inner member through the puncture and into a lumen of the vessel. The inner member can be pulled against the inner vessel surface and portions of a connecting member and a sealing membrane, which are coupled on their first ends to the inner member, can be arranged to extend to the outside of the vessel on their second ends. An outer member in a delivery configuration can then be inserted through the second end of the sealing membrane such that the sealing membrane at least partially surrounds the outer member. A compressive force can be applied to the outer member in a distal direction to expand the delivery configuration to a sealing configuration. This expansion can urge a mid-region of the sealing membrane radially outward such that its outer surface contacts and conforms to an edge of the hole.

### Definitions:

Certain terms are used throughout this patent document to refer to particular features or components. As one skilled in the art will appreciate, different people may refer to the same feature or component by different names. This patent document does not intend to distinguish between components or features that differ in name but not in function. For the following defined terms, certain definitions shall be applied unless a different definition is given elsewhere in this patent document.

The terms "bioabsorbable," "biodegradable," and "bioresorbable" refer to the ability of a material to disintegrate or degrade so that no material remains after a predetermined period of time, such as after 1 week, after 5 years, or any period of time therebetween.

The terms "distal" and "proximal" refer to a position or direction relative to a treating clinician. "Distal" and "distally" refer to a position that is distant, or in a direction away, from the clinician. "Proximal" and "proximally" refer to a position that is closer to, or in a direction toward, the clinician.

The terms "inner surface" and "outer surface" refer to bodily tissue surfaces near a puncture or other opening. The term "inner surface" refers to an intra-luminal surface of a wall of a blood vessel or a wall of a body cavity. The term "outer surface" refers to an extra-luminal surface of a wall of a blood vessel or a wall of a body cavity. When the puncture or other opening is a septum between two body cavities or a biopsy tract, the "outer surface" is the surface proximal to a treating clinician, and the "inner surface" is the surface distal to the clinician.

The terms "patient" and "subject" refer to mammals and include both humans and animals.

All numeric values are assumed to be modified by the term "about," whether or not explicitly indicated. The term "about" refers to a range of numbers that one of skill in the art would consider equivalent to the recited value (e.g., having the same function or result). In many instances, the term "about" can include numbers that are rounded to the nearest significant figure.

The recitation of numerical ranges by endpoints includes all numbers and sub-ranges within and bounding that range (e.g., 1 to 4 includes 1, 1.5, 1.75, 2, 2.3, 2.6, 2.9, etc. and 1 to 1.5, 1 to 2, 1 to 3, 2 to 3.5, 2 to 4, 3 to 4, etc.).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

### Example applications of the present teachings:

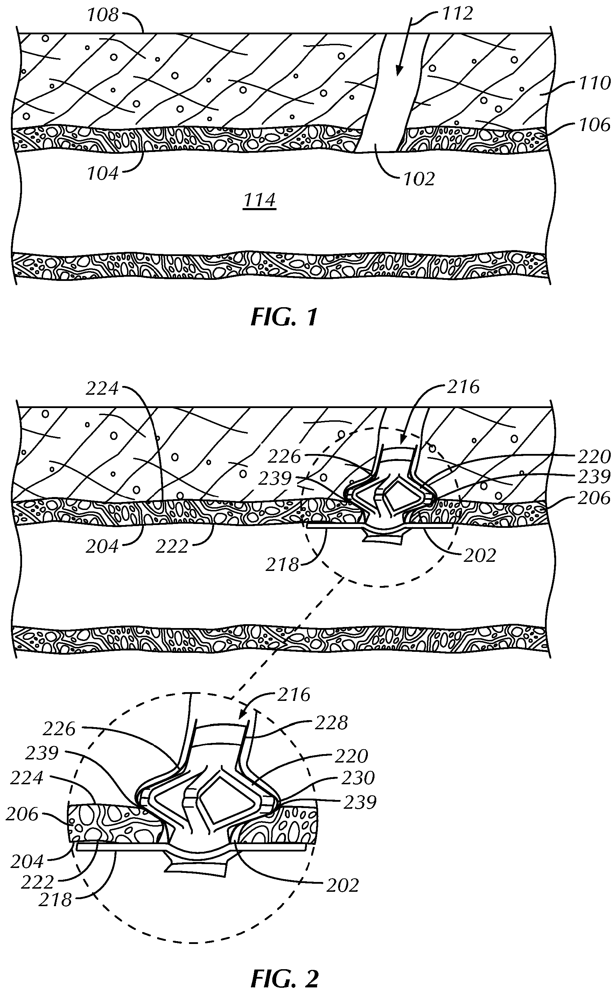

Vascular procedures are performed throughout the world and require access to a blood vessel of the vasculature system through a puncture. FIG. **1** is a schematic illustration of a puncture **102** in a wall **104** of a blood vessel **106** (e.g., a femoral artery). The vessel **106** is shown in cross-section passing beneath skin **108** and subcutaneous tissue **110** of a patient. The vessel **106** has been accessed by way of a percutaneous surgical procedure, which has resulted in an access path **112** consisting of a tissue tract and the puncture **102.** For example, the tract and puncture **102** may have resulted from inserting an introducer assembly into a lumen **114** of the vessel **106.**

The present closure systems can be used to seal a puncture or another opening in a vessel wall, body cavity, or biopsy tract that has been created intentionally or unintentionally during a surgical procedure. Punctures made intentionally include vascular punctures made in various types of vascular, endoscopic or orthopaedic surgical procedures, or punctures made in any other type of surgical procedure, in coronary or peripheral arteries and veins, or in a body cavity. Such procedures include angiographic examination, angioplasty, laser angioplasty, valvuloplasty, atherectomy, stent deployment, rotablator treatment, aortic prosthesis implantation, aneurysm repair, ventricular-assist device deployment, intraortic balloon pump treatment, pacemaker implantation, any intracardiac procedure, electrophysiological procedures, interventional radiology, and various other diagnostic, prophylactic, and therapeutic procedures such as dialysis and procedures relating to percutaneous extracorporeal circulation.

### Implant assembly:

FIG. **2** is a schematic illustration of an implant assembly **216** in its fully deployed configuration and sealing a puncture **202** in a wall **204** of a vessel **206,** as constructed in accordance with at least one embodiment. Sealing can be achieved by respectively applying inner **218** (intra-luminal) and outer **220** (extra-luminal) members of the implant assembly **216** against inner **222** and outer **224** vessel wall surfaces surrounding the puncture **202.** The inner member **218** can be pulled against the inner surface **222** of the vessel wall **204,** and the outer member **220** can be pushed against the outer surface **224** of the vessel wall **204** so that the puncture **202** is contained or sandwiched between the members. Alternatively, the outer member **220** can be deployed such that it expands between the inner **222** and outer **224** surfaces of the vessel wall **204** or at a position spaced from the outer surface **224** of the vessel wall **204.**

The implant assembly **216** can also include a sealing membrane **226** that extends from the inner member **218** and is configured at its proximal end **228** to receive the outer member **220,** which can expand to a sealing circumference larger than that of the puncture **202.** The presence of the sealing membrane **226** allows the implant assembly **216** to be used to seal various puncture sizes, including small, medium and large punctures resulting from 8F-24F introducer sheaths, for example. Based on the position of the outer member **220** within the sealing membrane **226** and the expandability of the outer member, the material of the sealing membrane in its mid-region **230** can be caused to expand and conform to edges **239** of the puncture **202.**

All components of the implant assembly **216** can be made to be bioabsorbable. This ensures that the implant assembly **216** is absorbed into the patient after a predetermined period of time that is sufficient to permit biological repair of the vessel wall **204** and the tissue around the puncture **202.** The implanted components should remain intact and slowly absorb or melt away, with no pieces coming loose and entering the blood stream. Further, it is desirable that the components initially maintain their strength and integrity so that the puncture **202** in the vessel wall **204** can begin to heal prior to the components beginning to weaken and absorb into the body.

Bioabsorbable polymers, such as polylactic acid (PLA), polyglycolic acid (PGA), trimethylene carbonate (TMC) and caprolactone (CL) can be used to form one or more of the components. Other suitable bioabsorbable polymers include, but are not limited to, poly D,L-lactide acid (PDLA), poly-L-lactic acid (PLLA), polyethylene glycol (PEG), polylactide-co-glycol acid (PLGA), polyanhydrides, polyorthoesters, polyethylene oxide (PEO), polydioxanone (PDS), polypropylene fumarate, polyethyl glutamate-co-glutamic acid, polytert-butyloxy-carbonylmethyl glutamate, polycaprolactone co-butylacrylate, polyhydroxybutyrate (PHBT) and copolymers of polyhydroxybutyrate, polyphosphazene, poly(D,L-lactide-co-caprolactone) (PLA/PCL), poly(glycolide-co-caprolactone) (PGA/PCL), polyphosphate ester, poly amino acid and polyhydroxy butyrate, polydepsipeptides, maleic anhydride copolymers, polyphosphazenes, polyiminocarbonates, poly[(97.5% dimethyl-trimethylene carbonate)-co-(2.5% trimethylene carbonate)], cyanoacrylate, polycyanoacrylates, polyethylene oxide, hydroxypropylmethylcellulose, polysaccharides (such as hyaluronic acid, chitosan and regenerate cellulose), and proteins (such as gelatin and collagen). These polymers can have a structure this is amorphous and can include a glass transition temperature that is close to the natural temperature of the body.

The components of the implant assembly **216** can optionally be designed to provide the strength and absorbtion rate desired through the use of material combinations, over-molding or other coating techniques. The entire implant assembly **216** or specific portions of one or more of its components can be over molded, coated, or otherwise incorporate a second, third, etc. material to alter its strength or absorbtion profile. In an example, the second material can be an alkaline earth metal, such as magnesium. It is believed that the combination of a magnesium material and an acidic polymer (e.g., PLA) can allow for tailoring the speed of component decay. It has been observed that magnesium does not dissolve in an environment having a pH above about 8, but degrades relatively rapidly at a pH of about 7.4 to 7.6. The acidic nature of the polymer can maintain the pH in the vicinity of the magnesium below a critical level, such as below 8, thereby encouraging degradation.

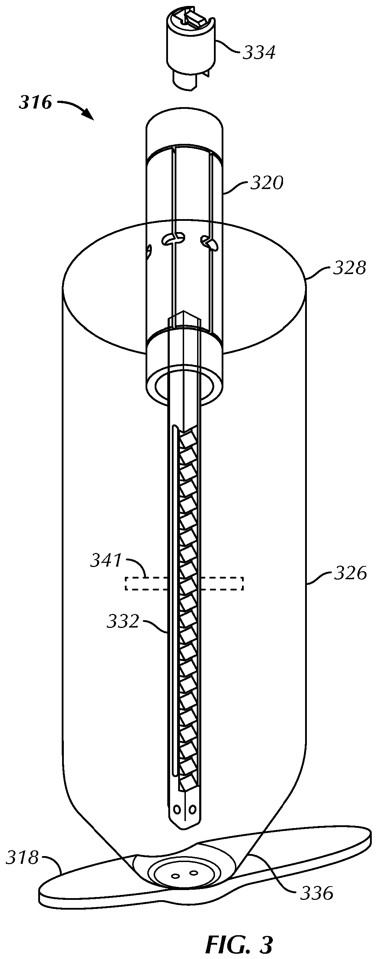

FIG. 3 is an exploded view of an implant assembly **316,** as constructed in accordance with at least one embodiment. The implant assembly **316** can comprise three primary components, namely, an inner member **318,** a sealing membrane **326** and an outer member **320.** The implant assembly **316** can further comprise a connecting member **332,** which can optionally be incorporated into the inner member **318,** and a locking member **334,** which can optionally be incorporated into the outer member **320.**

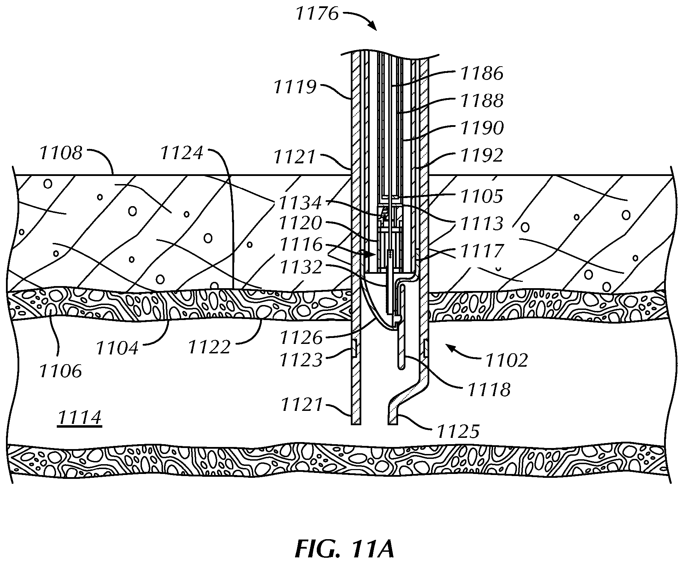

In use, the components of the implant assembly **316** can be placed and assembled in a distal-to-proximal manner (see, e.g., FIGS. **11A-11C****).** The inner member **318** can first be inserted through a puncture in a wall of a vessel and introduced into a lumen of the vessel. The inner member **318** can then be retracted until it is in contact with an inner surface of the vessel wall. The connecting member **332** and the sealing membrane **326** can extend through the puncture, and the connecting member **332** can be used to hold the inner member **318** against the inner surface of the vessel wall. The outer member **320**-illustrated in its undeployed, delivery configuration-can then be received by the sealing membrane **326**-which can be in the form of a generally elongated tubular body with a closed or sealed distal end **336** and an open proximal end **328-**and advanced along the connecting member **332** until it contacts the outer surface of the vessel wall or until it contacts a stop member **341** placed along the length of the connecting member **332.** The stop member **341** can provide a predetermined stop of the outer member **320** for closure of punctures such as apical access for mitral valve replacement in which there is a relatively long path to seal. Tension can be maintained on the connecting member **332** and a pushing force can be applied to the outer member **320** to expand its delivery configuration to a sealing configuration. Once the outer member **320** has achieved a longitudinally-compressed and transversely-expanded state, the locking member **334** can be secured to the connecting member **332.** In other examples, the proximal end **328** can have a closed or sealed proximal end **328** and the outer member **320** can be urged against the closed end.

### Inner member and connecting member of implant assembly:

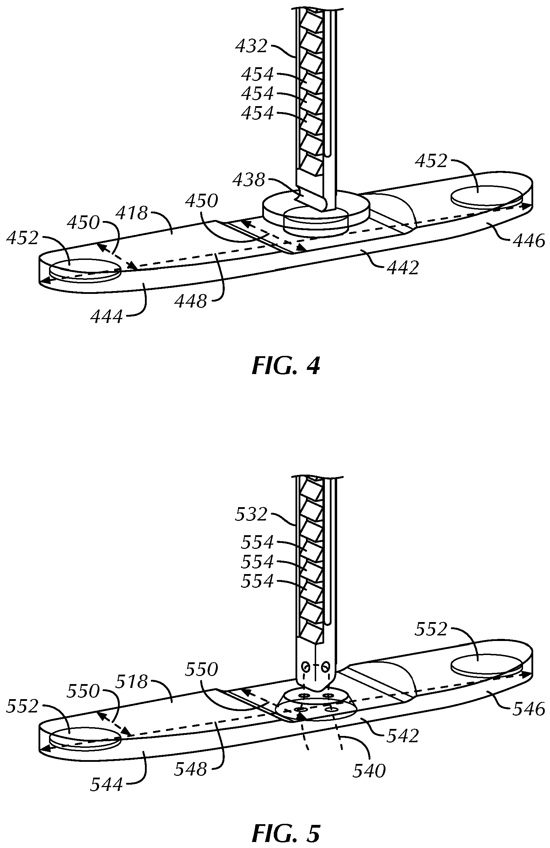

FIGS. **4** and **5** are schematic illustrations of an inner member **418, 518** and a connecting member **432, 532,** as constructed in accordance with at least two embodiments. The connecting member **432, 532** can be a stem, rod, tube, string, thread or other filament that is part of the inner member **418, 518,** or can be a stem, rod, tube, string, thread or other filament that is attached to the inner member **418, 518.** FIG. **4** illustrates a configuration in which the inner member **418** and the connecting member **432** are combined into a single molded piece. A hinge **438** can be incorporated at the base of the connecting member **432** to allow the connecting member to bend or otherwise reorientate with respect to the inner member **418.** This bending can be beneficial when delivering the inner member **418** through an introducer sheath and puncture, as illustrated in FIG. **11A****.** In an example, the hinge **438** can be formed by notching the base of the connecting member **432.** FIG. **5** illustrates a configuration in which the inner member **518** and the connecting member **532** are separate, but attachable pieces. In an example, the connecting member **532** can be attached to the inner member **518** using a dissolvable suture **540,** a mechanical fastener, adhesive or any other suitable joining means. When formed separately, the inner **518** and connecting **532** members may or may not be comprised of the same bioabsorbable material or material combination.

The inner member **418, 518** can be configured to be extended through a puncture in a wall of a vessel and into a lumen of the vessel to at least partially block the internal opening of the puncture. As illustrated in FIGS. **4** and **5****,** the inner member **418, 518** can have an enlarged central region **442, 542,** positioned between first **444, 544** and second **446, 546** end regions, where it attaches to the connecting member **432, 532.** This enlarged region **442, 542** can provide a sufficient area to cover the internal opening of the puncture. A longitudinal axis **448, 548** can define a lengthwise dimension and transverse axes **450, 550** can define widthwise dimensions. The lengthwise dimension can range from about 10mm to about 20mm, for example. The widthwise dimension at the end regions can range from about 3mm to about 5mm, for example. At the enlarged region **442, 542,** the widthwise dimension can progressively increase so that its maximum width ranges from about 4mm to about 8mm, for example.

The inner member **418, 518** can be composed of a thin, narrow strip or bar of material that is sufficiently rigid to be resistant to deformation, yet thin enough to conform to the inner surface of the vessel wall and not take up a substantial portion of the lumen of the vessel. It can be important for the inner member **418, 518** to be resistant to deformation to preclude it from bending and passing back through the puncture in which it was introduced. It can also be important that in its final *in situ* position, as illustrated in FIG. **2****,** the inner member **418, 518** does not block or otherwise impede the flow of blood through the vessel. Since the component can be formed of a resorbable material, it can be left in pace within the body until it is absorbed.

The inner member **418, 518** can be asymmetrically-shaped and include radiopaque marking elements **452, 552** to help a treating clinician position it within a vessel. For example, the inner member **418, 518** can include a first end region **444, 544** that is longer than a second end region **446, 546** to encourage pivoting about its attachment location to the connecting member **432, 532** when deployed from an introducer sheath. In the embodiment shown, the first end region **444, 544** can be about 1/3 longer than the second end region **446, 546.** The inner member **418, 518** can also include the radiopaque marking elements **452, 552** or a coating that is viewable on x-ray making visualization during delivery and deployment possible. The radiopaque material can be sodium diatrizoate, iopamidol, iohexol, iodixanol, iopromide, or another water soluble material, for example, and can be added to the material or material combination from which the inner member is composed or encapsulated in one or more pockets therein. In an example, the radiopaque marking elements **452, 552** are encapsulated within each of the first **444, 544** and second **446, 546** end regions of the inner member **418, 518.** In other examples, the radiopaque material can be non-water soluble, such as tungsten and platinum, and in the form of particles, washers, or wires (longitudinal or circumferential).

The connecting member **432, 532** can have a length and cross-sectional size that allows it to fit through the puncture of the vessel wall. It can be desirable for the connecting member to have a length of at least about 38mm (pre-cut) and at least about 9mm (post-cut), such as between about 5mm and about 60mm, for example. The connecting member **432, 532** can have a constant or varying cross-sectional size. The connecting member **432, 532** can have a perpendicular or angled orientation relative to the inner member **418, 518** in the absence of external forces (e.g., delivery forces from a wall of an introducer sheath).

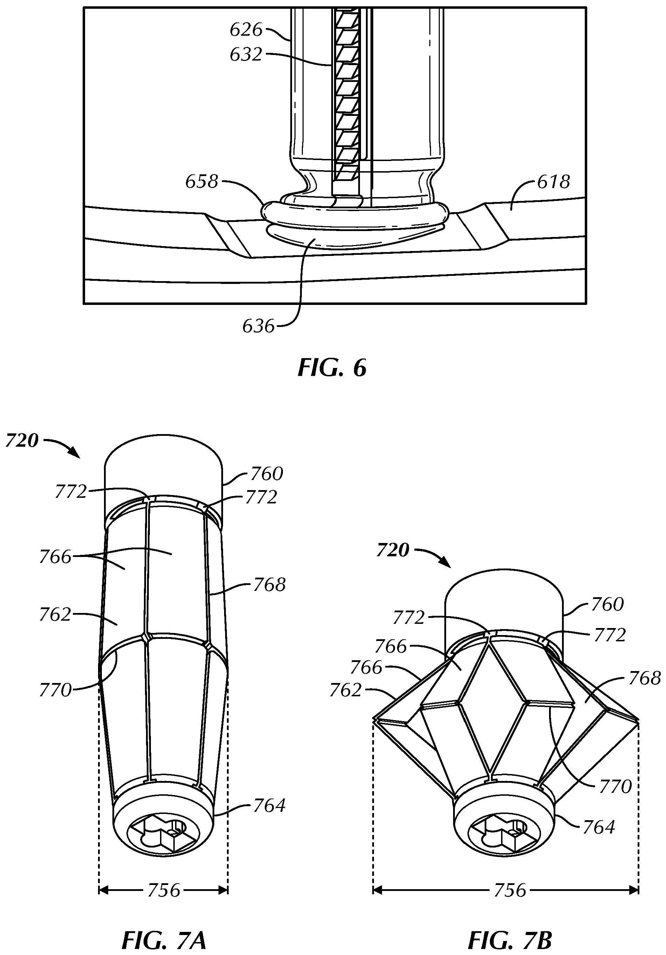

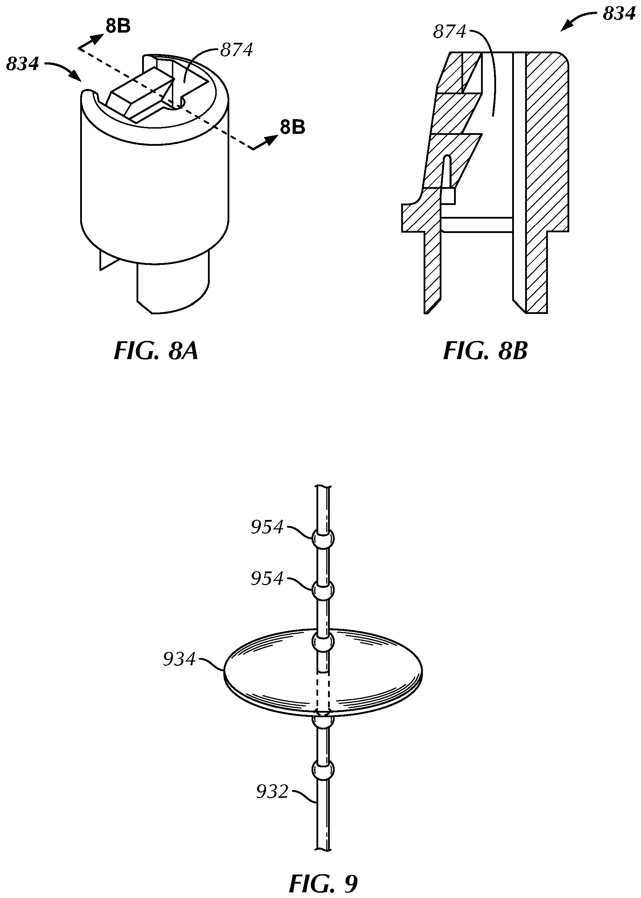

A number of pawls **454, 554** or other spaced elements can be formed on an outer surface of the connecting member **432, 532.** The pawls **454, 554** can be spaced from each other in the longitudinal direction of the connecting member **432, 532.** The presence of these spaced pawls **454, 554** can allow for adjusting the distance between a proximal end of an outer member (see, e.g., FIGS. **7A** and **7B**) and a proximal end of the inner member **418, 518.** For example, during deployment of an implant assembly at a puncture site, the outer member can be pushed against the outer surface of the vessel wall or a stop member (see, e.g., element **341** of FIG. **3**) by urging the proximal end of the outer member in a distal direction toward the proximal end of the inner member **418, 518.** A locking member (see, e.g., FIGS. **8A** and **8B**) can provide resistance and prevent the proximal end of the outer member from retracting proximally and disengaging from the vessel wall or stop member.

### Sealing membrane of implant assembly:

FIG. **6** is a schematic illustration of a sealing membrane **626** attached to an inner member **618** on its distal end **636** and surrounding portions of a connecting member **632** along its mid-region and proximal end portions, as constructed in accordance with at least one embodiment. In use, the distal end **636** of the sealing membrane **626** can be located within or near an inner surface of the puncture, while the proximal end can be located outside of an outer surface of the puncture and can include an opening through which an outer member can be received. When using the inner member embodiment illustrated in FIG. **4****,** the distal end of the sealing membrane can be slid over a boss on an upper surface of the inner member **618** and pinched in an undercut section using an absorbable suture or O-ring **658,** for example. When using the inner member embodiment illustrated in FIG. **5****,** the distal end **636** of the sealing membrane **626** can be inserted through a center hole of the inner member **618** and pinched in placed by a tapered plug member. Alternatively or conjunctively, the sealing membrane **626** can be secured to the inner member **618** using a medical-grade adhesive, thermal or solvent bonding, or any other suitable joining means.

The sealing membrane **626** can be formed of a flexible, fluid impermeable and biodegradable polymer having an inner diameter or about 2mm or more, a wall thickness in a range of about 0.025mm to about 0.38mm, and which can be cut to have a length of at least about 10mm, for example. In an example, the sealing membrane **626** is formed of STRATAPRENE^{®} 3534 copolymer material, which is commercially available from Poly-Med, Inc., of Anderson, South Carolina. Because the sealing membrane **626** can be constructed from a flexible material, it is capable of expanding and contracting to accommodate changes in the size and shape of the outer member (e.g., during its change from a delivery configuration to a sealing configuration). In addition, because the outer member is larger than the diameter of the puncture in its deployed, sealing configuration, the mid-region of the sealing membrane **626** can continuously remain in contact with edges of the puncture and prevent blood leakage.

### Outer member of implant assembly:

FIGS. **7A** and **7B** are schematic illustrations of an outer member **720** that can be expanded from a delivery configuration (FIG. **7A**) to a sealing configuration (FIG. **7B**), as constructed in accordance with at least one embodiment. The low profile of the delivery configuration can be useful to facilitate delivery of the outer member **720,** while the sealing configuration can be useful to facilitate firmly lodging the outer member **720** in a tissue tract and sealing a puncture against outward blood flow. In an example, a diameter **756** of the outer member **720** in the sealing configuration (FIG. **7B**) is at least three times the diameter **756** of the outer member **720** in the delivery configuration (FIG. **7A**). This can allow the outer member **720,** when in the sealing configuration, to cover the puncture and neighboring portions of the puncture from outside of the blood vessel wall.

The outer member **720** can be formed from a substantially rigid body with a generally cylindrical or polygonal (e.g., square or hexagonal) shape and having a proximal end **760,** an intermediate deformation portion **762,** and a distal end **764.** The body can have a pre-expanded, delivery length between about 6mm and about 20mm, and a pre-expanded, delivery diameter between about 2.5mm and 20mm, for example. Other shapes and dimensions are also possible.

The intermediate deformation portion **762** of the outer member **720** can include support struts **766** created by parallel slits or cuts **768** completely or partially through the wall of the member. The center portions of the support struts **766** can move radially outward in a hinge-like manner in response to the movement of the member's proximal end **760** toward its distal end **764.** In the delivery configuration, the struts **766** of the intermediate deformation portion **762** can be elongated in a direction substantially perpendicular to an *in situ* inner member, while in the sealing configuration, the struts **766** can be contracted in a direction substantially parallel to the *in situ* inner member. A hinge **770** near the center of the struts **766** can be formed in a variety of ways, including mechanical thinning, denting, grinding, heat forming or machining, or a weakened section created by micro cuts or tapered grooves. The number, length and distance between the slits or cuts **768** can affect both the anchorability of the outer member **720** and the collapsibility of the member. As an alternative to parallel slits or cuts **768,** the slits or cuts can be helical or serpentine such that they are adapted to expand and form wings upon rotation of the member's proximal end **760** relative to its distal end **764.**

The slits or cuts **768** in the intermediate deformation portion **762** of the outer member **720** can be formed using an etching or cutting process. For example, the slits can be cut along the intermediate portion of the rigid body using a cutting tool, such as a razor blade. According to some embodiments, the slits are cut without removing any significant amount of material from the rigid body, i.e., the formation of the slits does not significantly reduce the overall volume of rigid body. According to other embodiments, the slits are formed by cutting material out of the rigid body such that its volume is reduced.

One or both ends **772** of each slit **768** can be rounded so-as-to relieve stresses at the axial ends of the slit. This can prevent the slits from lengthening due to stress. In embodiments where the slits are cut without removing any significant amount of material from the rigid body, rounded ends can be produced by burning holes at both ends of each slit. In embodiments where the slits are formed by cutting material out of the rigid body, rounded ends can be formed during the cutting process. The size of the rounded ends can vary depending upon the dimensions of the rigid body and the amount of stress release required.

The outer member **720** can be disposed within the lumen of a sealing membrane and received over and slid along a connecting member so that the distance between the outer member and the inner member is adjustable in an aligned manner, depending on a puncture or opening to be sealed. This makes it possible to cope with a variety of situations or cases, such as a patient with a thick, thin, hard or soft *in vivo* vessel wall. During expansion of the outer member **720** to its sealing configuration, the sealing membrane can exert counteractive or countervailing contractile forces on the outer member. In this way, the sealing membrane does not expand passively, but rather, the outer member **720** forcibly expands the sealing membrane to cause the membrane to be taunt and capable of sealing the puncture in a fluid tight manner. Alternatively, the sealing membrane can be sized and shaped such that the outer member **720** expands to its sealing configuration to fill the space within the sealing membrane.

Although not shown, it is contemplated that more than one outer member **720** can be used, such as in a stacking arrangement. In such an example, the expanded diameters of the outer members **720** can decrease in size as the distance from the treatment site (e.g., vessel wall puncture) increases.

### Locking member of implant assembly:

FIGS. **8A, 8B** and **9** are schematic illustrations of a locking member **834, 934** that can be received by a proximal end of a connecting member **932,** as constructed in accordance with at least two embodiments. The locking member **834, 934** can be slid along the connecting member and into abutment against a proximal end of an outer member, thereby maintaining an *in situ* position of an inner member against an inner surface of a vessel wall, an expanded configuration of an outer member against an outer surface of the vessel wall or a stop member (see, e.g., element **341** of FIG. **3**), and contact between a sealing membrane and edges of a puncture (as illustrated in FIG. **2**) or tissue tract. Portions of the connecting member proximal to the locking member can be cut using a mechanical means (e.g., shearing arrangement) or thermal means and removed after the locking member **834, 934** has been secured to the connecting member.

The locking member **834, 934** can take a variety of forms, non-limiting examples of which are provided in FIGS. **8A, 8B** and **9****.** One skilled in the art will recognize that the locking member **834, 934** can assume numerous other configurations while retaining its capability to maintain a position of the inner and outer members. FIGS. **8A** and **8B** illustrate a configuration in which the locking member **834** comprises a notched passageway **874** that is slidable and receives a number of pawls or other spaced elements formed on an outer surface of the connecting member in one direction, but is resistant to sliding of the pawls or other spaced elements in a second, opposite direction. FIG. **9** illustrates a configuration in which the locking member **934** comprises a compressible disk that is slidable and receives a connecting member **932** in the form of a filament having a plurality of ball projections **954** thereon. Upon the application of sufficient pulling force to the filament, one or more ball projections **954** can pass through a central lumen of the disk to lock a position of the filament.

While FIGS. **8A, 8B** and **9** illustrate distinct locking members, the locking member can optionally be integrated into a portion of the outer member, such as at its proximal end.

### Delivery assembly:

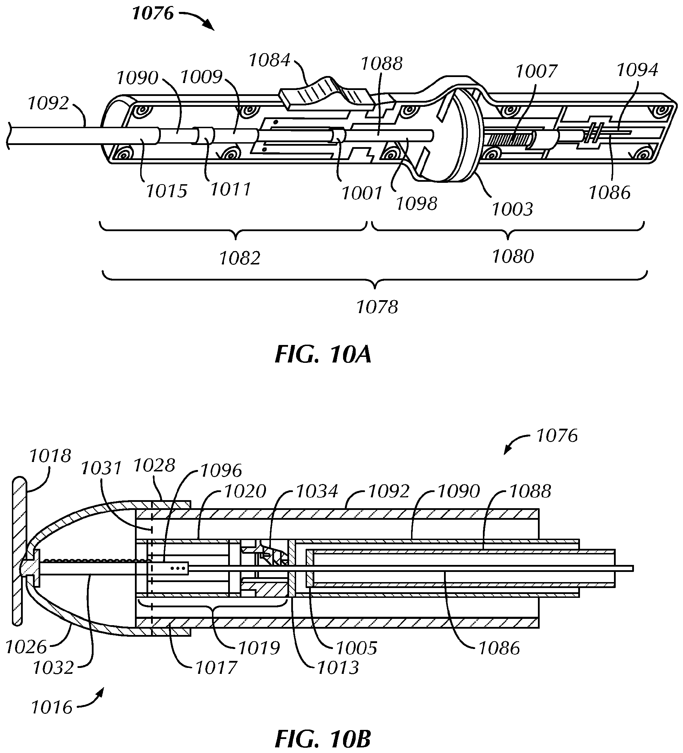

FIG. **10A** is a schematic cross-sectional illustration of a proximal portion of a delivery assembly **1076** configured to deploy an implant assembly at a puncture site, as constructed in accordance with at least one embodiment. FIG. **10B** is a schematic cross-sectional illustration of an implant assembly **1016** mounted on a distal portion of the delivery assembly **1076,** as constructed in accordance with at least one embodiment.

The delivery assembly **1076** can extend from a handle **1078,** operable by a treating clinician, at its proximal portion to connections with components of the implant assembly **1016** at its distal portion. The handle **1078** can include a proximal housing portion **1080** and a distal housing portion **1082.** A handle lock **1084,** which can be activated during initial delivery steps, prevents relative movement between the two housing portions **1080, 1082.** When the lock **1084** is not activated, the two housing portions **1080, 1082** can be rotated relative to one another.

Four elongate members can extend distally from the handle **1078** to the implant assembly **1016,** namely (moving from inside-out), a rail **1086,** a shear tube **1088,** a push tube **1090,** and a delivery tube **1092.**

The rail **1086** and the shear tube **1088** can be engaged with the proximal housing portion **1080** on their proximal ends. The rail **1086** can extend from a proximal end **1094,** which is statically coupled with the proximal housing portion **1080,** to a distal end **1096,** which is engaged with an inner member **1018** of the implant assembly **1016.** In an example, the rail **1086** can include a stainless steel ribbon having a rectangular cross-section. The proximal end of the ribbon can be secured in a rectangular pocket in the proximal housing portion **1080.** The distal end of the ribbon can be molded into a proximal end of a connecting member **1032** of the implant assembly **1016.** The connecting member **1032** attaches to the inner member **1018** on its distal end. The connecting member **1032** can include a rectangular cross-section slightly larger than that of the rail **1086.** The shear tube **1088** can extend from a proximal end **1098,** which is engaged with an axial tract **1001** and an actuation member **1003** of the proximal housing portion **1080,** to a distal end **1005,** which includes a keyed passageway and abuts locking **1034** and outer **1020** members of the implant assembly **1016.** The axial tract **1001** can ensure that the shear tube **1088** can only move axially relative to the proximal housing portion **1080,** with such axial movement being created through movement of the actuation member **1003.** In an example, the actuation member **1003** includes a rotatable knob engaged with a threaded shaft **1007.** Rotation of the knob in a first direction urges distal movement of the threaded shaft **1007.** The distal movement of the threaded shaft **1007** causes distal movement of the shear tube **1088,** which can transfer such movements (via push tube **1090**) to the locking **1034** and outer **1020** members of the implant assembly **1016.**

The push tube **1090** and the delivery tube **1092** can be engaged with the distal housing portion **1082** on their proximal ends. The push tube **1090** can extend from a proximal end **1009,** which is engaged with an axial tract **1011** of the distal housing portion **1082** such that the tube can only move axially relative to the distal housing portion, to a distal end **1013,** which includes a keyed passageway similar in size and shape to the keyed passageway of the shear tube **1088.** The push tube **1090** surrounds portions of the shear tube **1088** and its distal end **1013** extends distal to the distal end **1005** of the shear tube. As such, when the shear tube **1088** is moved axially in a distal direction, the push tube **1090** is also moved distally. The keyed passageways of the shear **1088** and push **1090** tubes can be configured to receive and slide along the rail **1086** and the connecting **1032** member. The delivery tube **1092** can concentrically surround portions of the push tube **1090** and the shear tube **1088.** On its proximal end **1015,** the delivery tube **1092** can be statically coupled with the distal housing portion **1082;** on its distal end **1017,** an outer surface of the delivery tube **1092** can be statically coupled to a proximal end **1028** of a sealing membrane **1026** of the implant assembly **1016.** Prior to distal movement of the shear **1088** and push **1090** tubes, the distal end **1017** of the delivery tube **1092** can be positioned distal to the distal ends **1005, 1013** of the shear and push tubes, thereby creating an internal cavity **1019** for placement of the locking **1034** and outer **1020** members during initial delivery. After distal movement of the shear **1088** and push **1090** tubes, the distal end **1017** of the delivery tube **1092** can be positioned proximal to the distal ends **1005, 1013** of the shear and push tubes.

The handle **1078** can include certain features to ease manufacturing and use. For example, the handle **1078** can include a clamshell-like configuration including four pieces (two for each of the proximal and distal housing portions) allowing for the placement of proximal ends of the four elongate members during manufacture. A distal end of the handle **1078** can include engagement features (e.g., snap lock arms) configured to detachable couple to a proximal end of an introducer sheath. Further, an outer surface portion of the handle **1078** can include a flat surface, visible marking or other indicator means that conveys an orientation of the distally-positioned implant assembly **1016** to the treating clinician.

### Delivery of implant assembly using introducer sheath and delivery assembly:

FIGS. **11A-11C** are schematic illustrations, in sequential order, of an introducer sheath **1119** and a delivery assembly **1176** delivering and deploying an implant assembly **1116** at a puncture site **1102,** as constructed in accordance with at least one embodiment. The delivery assembly **1176** may take other suitable configurations, but will be described in association with the configuration illustrated and described in FIGS. **10A** and **10B****.** A dilator (not shown) can be inserted through the introducer sheath **1119** and over a guidewire (not shown) into a vessel lumen **1114** such that portions of the introducer sheath **1119** and dilator extend through an opening in a patient's skin **1108,** through a tissue tract, through the puncture **1102** and into the vessel lumen **1114.** The location of the introducer sheath's distal end **1121** can be verified using a radiopaque marker band **1123** embedded or otherwise attached to the sheath. Next, the guidewire and dilator can be withdrawn, and distal portions of the delivery assembly **1176** can be inserted into a proximal end of the introducer sheath **1119** using a valve bypass (not shown) and advanced toward the distal end **1121** of the introducer sheath, as illustrated in FIG. **11A****.** An inner member **1118** of the implant assembly **1176** can be retained in a longitudinal, axially-aligned orientation by a wall of the introducer sheath **1119** during this advancement.

The delivery assembly **1176** can be further advanced through the introducer sheath **1119** and past its distal tip **1121** so that the inner member **1118** extends into the vessel lumen **1114.** As the inner member **1118** is released from the confines of the introducer sheath **1119,** it can be pivoted to a transverse orientation by way of a dimple (or concavity) **1125** at the distal end **1121** of the introducer sheath. This pivoting of the inner member **1118** can be further encouraged by an offset connection to a connecting member **1132.** After the inner member **1118** is advanced past the distal end **1121** of the introducer sheath **1119** and into the vessel lumen **1114,** the introducer sheath **1119** can be pulled proximally and its proximal hub can be brought into engagement with snap lock arms on a distal end of a handle of the delivery assembly **1176.** This proximal pulling moves the distal end **1121** of the introducer sheath **1119** away from the vicinity of the vessel wall **1104,** providing space for components of the implant assembly **1116** to be deployed, and locks the sheath to the delivery assembly **1176** so that they can be simultaneously removed at the end of a closure procedure.

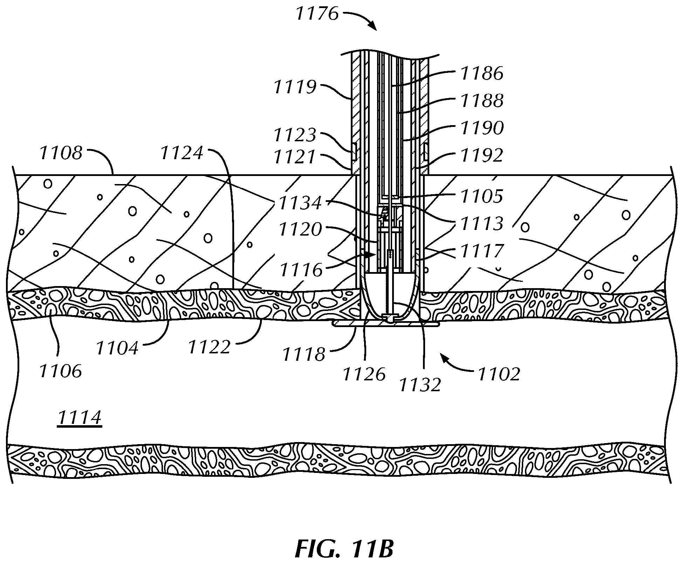

Next, as illustrated in FIG. **11B****,** the handle can be gently pulled proximally to urge the inner member **1118** against an inner surface **1122** of the vessel wall **1104** by way of a rail **1186** that can extend from a proximal housing portion to the connecting member **1132** engaged with the inner member **1118.** An enlarged region of the inner member **1118** can be positioned to span the internal opening of the puncture **1102.**

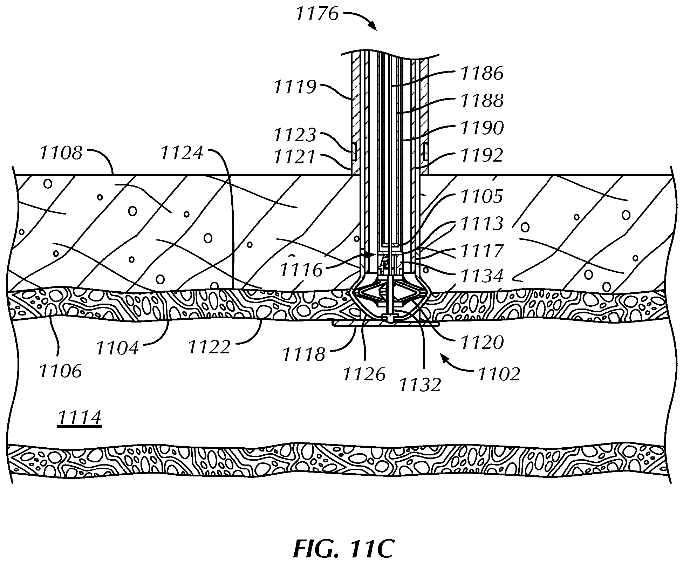

With the inner member **1118** in position, a knob on a proximal housing portion can be rotated to move distal ends **1105, 1113** of a shear tube **1188** and a push tube **1190** in a distal direction. This distal movement can urge an outer member **1120** and a locking member **1134** of the implant assembly from a cavity within a distal end **1117** of a surrounding delivery tube **1192,** along the rail **1186** and proximal portions of the connecting member **1132,** and through a proximal lumen of a sealing membrane **1126** of the implant assembly **1116** so that the distance between the outer **1120** and inner **1118** members is reduced. Additional rotation of the knob can cause the outer **1120** and locking **1134** members to move further along the connecting member **1132,** during which time a distal end of the outer member **1120** can contact an outer surface **1124** of the vessel wall **1104** (by way of the sealing membrane **1126),** and the outer member **1120** can be caused to expand against the surrounding sealing membrane **1126,** as illustrated in FIG. **11C****.** Recoil of this outer member expansion can be precluded by the abutting locking member **1134,** which can be engaged with the connecting member **1132.**

Portions of the connecting member **1132** proximal to the locking member **1134** can now be cut and removed. In an example, a lock associated with the handle can be unlocked to allow proximal and distal housing portions to be rotated relative to one another. Since the proximal end of the shear tube is rotatably secured to the proximal housing portion and the proximal end of the push tube is rotatably secured to the distal housing portion, relative rotation of the housing portions can cause corresponding relative rotation of the distal ends **1105, 1113** of the shear **1188** and push **1190** tubes and their associated keyed passageways. This relative rotation of the keyed passageways can shear the connecting member **1132** so that its proximal portions can be removed.

At this time, the only component remaining attached between the implant assembly **1116** and the delivery assembly **1076** is the sealing membrane **1126.** A distal end of the sealing membrane **1126** is attached to the inner member **1118,** can extend through portions of the puncture **1102** and surround portions of the outer member **1120,** and is attached to the distal end **1117** of the delivery tube **1192** at its proximal end (e.g., using UV adhesive, thermal bonding or solvent bonding). Leveraging perforations (see, e.g., element **1031** in FIG. **10B****)** in the sealing membrane **1126,** the handle can be pulled proximally to tear the sealing membrane **1126** along its perforations **1131** and separate the delivery tube **1192** from the implant assembly **1116.** Now separated from the implant assembly **1116,** the delivery assembly **1176** and the introducer sheath **1119** can be removed from the patient by further pulling the handle proximally, in a direction away from the patient.

### Closure system kit:

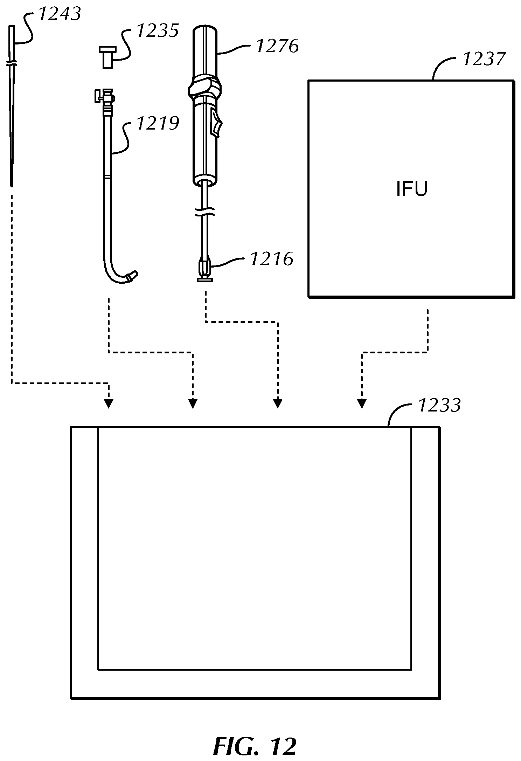

FIG. **12** is a schematic illustration of a kit **1233** including an implant assembly **1216,** a delivery assembly **1276,** an introducer sheath **1219** and a valve bypass **1235,** a dilator **1243,** and instructions **1237** for using these components to seal a puncture or other opening in a blood vessel wall, body cavity or biopsy tract, as constructed in accordance with at least one embodiment. The implant assembly **1216** can come preloaded and attached to distal end portions of the delivery assembly **1276.** In this way, a treating clinician, after gaining access to a vessel lumen with the dilator **1243** and introducer sheath **1219,** can perform the series of simple steps described and illustrated in FIGS. **11A-11C** to seal the puncture site. The dilator **1243** can be relatively long (e.g., about 80cm or more) and without a proximal hub to facilitate removal of a TAVI sheath or other interventional device, for example.

### Closing notes and examples:

The present closure systems can be used by a treating clinician to seal a puncture or other opening following removal of an introducer sheath or other interventional device, regardless of whether the device's profile is small, medium or large in size. The systems allow for closure of the puncture or opening using a construction that is easy to use, safe, effective and reliable. Implant components of a closure system can dissolve over a period of time while permitting healing of the puncture or opening and the surrounding tissue.

Although the figures and many embodiments relate to use of the present closure systems for sealing of a puncture associated with vascular surgery, one of ordinary skill in the art will appreciate that components disclosed herein are scalable and can be useful for closure of any puncture or other opening in a blood vessel wall, body cavity or biopsy tract of a patient. Further, it is contemplated that the present closure methods can be incorporated into another medical device such that cumulative delivery and deployment steps during a medical procedure are reduced.

The above Detailed Description includes references to the accompanying drawings, which form a part of the Detailed Description. The Detailed Description should be read with reference to the drawings. The drawings show, by way of illustration, specific embodiments in which the present systems can be practiced. These embodiments are also referred to herein as "examples."

The Detailed Description is intended to be illustrative and not restrictive. For example, the above-described examples (or one or more features or components thereof) can be used in combination with each other. Other embodiments can be used, such as by one of ordinary skill in the art upon reviewing the above Detailed Description. Also, various features or components have been or can be grouped together to streamline the disclosure. This should not be interpreted as intending that an unclaimed disclosed feature is essential to any claim. Rather, inventive subject matter can lie in less than all features of a particular disclosed embodiment.

## Claims

1. A closure system for a percutaneous puncture in a vessel wall, comprising:
a bioabsorbable inner member (418, 518) configured to overlay the percutaneous puncture and including an inner member body and one or more radiopaque elements (452, 552),
a sealing membrane (226, 326, 626, 1126);
an outer member (220, 320, 720, 1120);
the inner member body longitudinally extending from a first end to a second end and defining a length therebetween, and transversely extending from a first side to a second side and defining a width therebetween, wherein the width progressively increases between the first end and the second end to define an enlarged central region (442, 542) of maximum width positioned between first (444, 544) and second (446, 546) end regions,
the inner member body further having an upper surface configured to abut an inner surface of the vessel wall and an opposing lower surface,
a connecting member (332, 432, 532, 632, 932, 1032, 1132) operatively coupled to the inner member (418, 518) between the first end region (444, 544) and the second end region (446, 546), the connecting member (332, 432, 532, 632, 932, 1032, 1132) including a plurality of pawls (454, 554) formed on an outer surface thereof; and
the one or more radiopaque elements (452, 552) configured to enable visualization of the inner member body inside the vessel wall.

2. The closure system of claim 1, wherein the one or more radiopaque elements (452, 552) are water soluble.

3. The closure system of claim 1 or 2, wherein the one or more radiopaque elements (452, 552) are coated on the inner member body.

4. The closure system of claim 1, wherein the one or more radiopaque elements (452, 552) are within the inner member body.

5. The closure system of claim 1, wherein the first end region is about one-third (1/3) longer than the second end region.

6. The closure system of claim 5, wherein a first radiopaque element of the one or more radiopaque elements (452, 552) is encapsulated within the first end region and a second radiopaque element of the one or more radiopaque elements (452, 552) is encapsulated within the second end region.

7. The closure system of any one of claims 5 to 6, wherein the length of the inner member body ranges from about 10 mm to about 20 mm, inclusive, and the width at the enlarged region ranges from about 4 mm to about 8 mm, inclusive.

8. The closure system of any preceding claim, wherein the inner member body has a size and shape that does not block or otherwise impede a flow of blood through the vessel wall when the inner member abuts the inner surface of the vessel wall and overlays the percutaneous puncture.

9. The closure system of any preceding claim, wherein the connecting member is movably coupled to the inner member body.

10. The closure system of claim 9, wherein the connecting member is a stem, rod, tube, string, thread or other filament.

11. The closure system of claim 9 or 10, wherein the connecting member is movably coupled to the inner member body at a location closer to the second end of the inner member body than the first end of the inner member body.

12. The closure system of claim any one of claims 9 to 11, wherein the outer member (220, 320, 720, 1120) is expandable from a delivery configuration to a sealing configuration, the sealing configuration having a longitudinally-compressed and transversely-expanded state relative to the delivery configuration, wherein the outer member (220, 320, 720, 1120) is positionable adjacent an outer tissue surface relative to the inner member.

13. The closure system of claim 12, wherein the outer member (220, 320, 720, 1120) is bioabsorbable.

14. The closure system of claim 12 or 13, further comprising a locking member securable to the connecting member and configured to provide resistance to a proximal end of the outer member (220) from retracting proximally.

15. The closure system of claim 1, wherein the inner member body is composed of a thin strip or bar of rigid material.

## Patentansprüche

1. Verschlusssystem für eine perkutane Punktion einer Gefäßwand, umfassend:
ein bioresorbierbares inneres Element (418, 518), das so konfiguriert ist, dass es die perkutane Punktion überdeckt und einen inneren Elementkörper sowie ein oder mehrere röntgendichte Elemente (452, 552) beinhaltet,
eine Dichtungsmembran (226, 326, 626, 1126);
ein äußeres Element (220, 320, 720, 1120);
wobei sich der innere Elementkörper in Längsrichtung von einem ersten Ende zu einem zweiten Ende erstreckt und eine dazwischenliegende Länge definiert und sich in Querrichtung von einer ersten Seite zu einer zweiten Seite erstreckt und eine dazwischenliegende Breite definiert, wobei die Breite zwischen dem ersten Ende und dem zweiten Ende fortlaufend zunimmt, um einen vergrößerten Mittelbereich (442, 542) maximaler Breite zu definieren, der zwischen dem ersten (444, 544) und dem zweiten (446, 546) Endbereich positioniert ist,
wobei der innere Elementkörper ferner eine Oberseite, die an einer Innenfläche der Behälterwand anliegt, und eine gegenüberliegende Unterseite aufweist,
ein Verbindungselement (332, 432, 532, 632, 932, 1032, 1132), das zwischen dem ersten Endbereich (444, 544) und dem zweiten Endbereich (446, 546) mit dem inneren Element (418, 518) wirksam verbunden ist, wobei das Verbindungselement (332, 432, 532, 632, 932, 1032, 1132) eine Vielzahl von Sperrklinken (454, 554) beinhaltet, die an seiner Außenfläche ausgebildet sind; und
ein oder mehrere röntgendichte Elemente (452, 552), die so konfiguriert sind, dass sie Visualisierung des inneren Elementkörpers innerhalb der Gefäßwand ermöglichen.

2. Verschlusssystem nach Anspruch 1, wobei das eine oder die mehreren röntgendichten Elemente (452, 552) wasserlöslich sind.

3. Verschlusssystem nach Anspruch 1 oder 2, wobei das eine oder die mehreren röntgendichten Elemente (452, 552) auf dem inneren Elementkörper aufgebracht sind.

4. Verschlusssystem nach Anspruch 1, wobei sich das eine oder die mehreren röntgendichten Elemente (452, 552) innerhalb des inneren Elementkörpers befinden.

5. Verschlusssystem nach Anspruch 1, wobei der erste Endbereich etwa ein Drittel (1/3) länger als der zweite Endbereich ist.

6. Verschlusssystem nach Anspruch 5, wobei ein erstes röntgendichtes Element des einen oder der mehreren röntgendichten Elemente (452, 552) im ersten Endbereich eingeschlossen ist und ein zweites röntgendichtes Element des einen oder der mehreren röntgendichten Elemente (452, 552) im zweiten Endbereich verkapselt ist.

7. Verschlusssystem nach einem der Ansprüche 5 oder 6, wobei die Länge des inneren Elementkörpers im Bereich von etwa 10 mm bis etwa 20 mm liegt und die Breite im erweiterten Bereich im Bereich von etwa 4 mm bis etwa 8 mm liegt.

8. Verschlusssystem nach einem vorstehenden Anspruch, wobei der innere Elementkörper eine Größe und Form aufweist, die eine Blutfluss durch die Gefäßwand nicht blockiert oder anderweitig behindert, wenn das innere Element an der Innenfläche der Gefäßwand anliegt und die perkutane Punktion überdeckt.

9. Verschlusssystem nach einem vorstehenden Anspruch, wobei das Verbindungselement beweglich mit dem inneren Elementkörper verbunden ist.

10. Verschlusssystem nach Anspruch 9, wobei das Verbindungselement ein Stiel, eine Stange, ein Rohr, eine Schnur, ein Faden oder ein anderes Filament ist.

11. Verschlusssystem nach Anspruch 9 oder 10, wobei das Verbindungselement an einer Stelle, die näher am zweiten Ende des inneren Elementkörpers als am ersten Ende des inneren Elementkörpers liegt, beweglich mit dem inneren Elementkörper verbunden ist.

12. Verschlusssystem nach einem der Ansprüche 9 bis 11, wobei das äußere Element (220, 320, 720, 1120) von einer Abgabekonfiguration in eine Dichtungskonfiguration expandierbar ist, wobei die Dichtungskonfiguration im Vergleich zur Abgabekonfiguration einen längskomprimierten und querexpandierten Zustand aufweist, und wobei das äußere Element (220, 320, 720, 1120) relativ zum inneren Element an einer äußeren Gewebeoberfläche positionierbar ist.

13. Verschlusssystem nach Anspruch 12, wobei das äußere Element (220, 320, 720, 1120) bioresorbierbar ist.

14. Verschlusssystem nach Anspruch 12 oder 13, ferner umfassend ein Verriegelungselement, das an dem Verbindungselement befestigt werden kann und so konfiguriert ist, dass es einem proximalen Ende des äußeren Elements (220) Widerstand gegen ein proximales Zurückziehen bietet.

15. Verschlusssystem nach Anspruch 1, wobei der innere Elementkörper aus einem dünnen Streifen oder Stab aus starrem Material gebildet ist.

## Revendications

1. Système de fermeture pour perforation percutanée de la paroi d'un vaisseau, comprenant :
un élément interne bioabsorbable (418, 518) configuré pour recouvrir la perforation percutanée et incluant un corps d'élément interne et un ou plusieurs éléments radio-opaques (452, 552),
une membrane d'étanchéité (226, 326, 626, 1126) ;
un élément externe (220, 320, 720, 1120) ;
le corps de l'élément interne s'étendant longitudinalement depuis une première extrémité jusqu'à une seconde extrémité et définissant une longueur entre elles et s'étendant transversalement depuis un premier côté jusqu'à un second côté et définissant une largeur entre eux, dans lequel la largeur augmente progressivement entre la première extrémité et la seconde extrémité pour définir une région centrale élargie (442, 542) de largeur maximale positionnée entre la première (444, 544) et la seconde (446, 546) régions d'extrémité,
le corps de l'élément interne présentant en outre une surface supérieure configurée pour venir en butée contre une surface intérieure de la paroi du vaisseau et une surface inférieure opposée,
un élément de raccordement (332, 432, 532, 632, 932, 1032, 1132) couplé fonctionnellement à l'élément interne (418, 518) entre la première zone d'extrémité (444, 544) et la seconde zone d'extrémité (446, 546), l'élément de raccordement (332, 432, 532, 632, 932, 1032, 1132) incluant une pluralité de cliquets (454, 554) formés sur sa surface extérieure ; et
un ou plusieurs éléments radio-opaques (452, 552) configurés pour permettre la visualisation du corps de l'élément interne à l'intérieur de la paroi du vaisseau.

2. Système de fermeture selon la revendication 1, dans lequel les un ou plusieurs éléments radio-opaques (452, 552) sont solubles dans l'eau.

3. Système de fermeture selon la revendication 1 ou 2, dans lequel les un ou plusieurs éléments radio-opaques (452, 552) sont revêtus sur le corps de l'élément interne.

4. Système de fermeture selon la revendication 1, dans lequel les un ou plusieurs éléments radio-opaques (452, 552) sont à l'intérieur du corps de l'élément interne.

5. Système de fermeture selon la revendication 1, dans lequel la première région d'extrémité mesure environ un tiers (1/3) de plus que la seconde région d'extrémité.

6. Système de fermeture selon la revendication 5, dans lequel un premier élément radio-opaque parmi les un ou plusieurs éléments radio-opaques (452, 552) est encapsulé dans la première région d'extrémité et un deuxième élément radio-opaque parmi les un ou plusieurs éléments radio-opaques (452, 552) est encapsulé dans la seconde région d'extrémité.

7. Système de fermeture selon l'une quelconque des revendications 5 à 6, dans lequel la longueur du corps de l'élément interne varie d'environ 10 mm à environ 20 mm inclus, et la largeur au niveau de la région élargie varie d'environ 4 mm à environ 8 mm inclus.

8. Système de fermeture selon une quelconque revendication précédente, dans lequel le corps de l'élément interne présente une taille et une forme qui ne bloquent ni n'entravent autrement le flux sanguin à travers la paroi du vaisseau lorsque l'élément interne est en butée avec la surface interne de la paroi du vaisseau et recouvre la perforation percutanée.

9. Système de fermeture selon une quelconque revendication précédente, dans lequel l'élément de raccordement est couplé de manière mobile au corps de l'élément interne.

10. Système de fermeture selon la revendication 9, dans lequel l'élément de raccordement est une tige, un arbre, un tube, une ficelle, un fil ou un autre filament.

11. Système de fermeture selon la revendication 9 ou 10, dans lequel l'élément de raccordement est couplé de manière mobile au corps de l'élément interne au niveau d'un emplacement plus proche de la seconde extrémité du corps de l'élément interne que de la première extrémité du corps de l'élément interne.

12. Système de fermeture selon l'une quelconque des revendications 9 à 11, dans lequel l'élément externe (220, 320, 720, 1120) est apte à être déployé depuis une configuration de distribution jusqu'à une configuration d'étanchéité, la configuration d'étanchéité présentant un état comprimé longitudinalement et dilaté transversalement par rapport à la configuration de distribution, dans lequel l'élément externe (220, 320, 720, 1120) est positionnable près d'une surface de tissu externe par rapport à l'élément interne.

13. Système de fermeture selon la revendication 12, dans lequel l'élément externe (220, 320, 720, 1120) est bioabsorbable.

14. Système de fermeture selon la revendication 12 ou 13, comprenant en outre un élément de verrouillage pouvant être fixé à l'élément de raccordement et configuré pour fournir une résistance à une extrémité proximale de l'élément externe (220) afin d'éviter qu'il ne se rétracte de manière proximale.

15. Système de fermeture selon la revendication 1, dans lequel le corps de l'élément interne est composé d'une bande ou d'une barre mince de matériau rigide.
